# EUROPEAN PATENT APPLICATION

(11) **EP 1 256 346 A1**
(43) Date of publication of application: **13.11.2002**
(21) Application number: 00981742.0
(22) Date of filing: 15.12.2000
(51) Int. Cl.: A61K 31/60, A61P 17/00, A61P 37/08, A61K 9/06

(54) **REMEDIES FOR EXTERNAL USE FOR ALLERGIC SKIN DISEASES**

(30) Priority: 28.12.1999 JP 37355199
(71) Applicant: TEIKOKU SEIYAKU CO., LTD., Kagawa 769-2695 (JP)
(72) Inventor: INAMOTO, Yukiko, Kagawa-gun, Kagawa 761-1703 (JP); KAWATA, Mitsuhiro, Okawa-gun, Kagawa 769-2702 (JP); KAWABATA, Seiichiro, Okawa-gun, Kagawa 769-2601 (JP); NAKAYAMA, Daisuke, Takamatsu-shi, Kagawa 761-0104 (JP); HISAICHI, Shin-ichi, Kita-gun, Kagawa 761-0612 (JP); TOKUDA, Masaaki, Takamatsu-shi Kagawa 761-8071 (JP)
(74) Representative: Albrecht, Thomas, Dr.
(86) International application number: JP0008889
(87) International publication number: WO01047526

(57) **Abstract**

External preparations for allergic dermatitis containing Aspirin alone as the active ingredient, which exert an excellent therapeutic effects on allergic dermatitis with lower side effects; and a method for treating allergic dermatitis by using these agents for external use.

## Description

### Technical Field

The present invention relates to external preparations for treating allergic dermatitis and a method for treating allergic dermatitis. In more detail the present invention relates to external preparations for treating allergic dermatitis containing acetylsalicylic acid as the sole active ingredient and a method for treating allergic dermatitis by using an external preparation containing acetylsalicylic acid as the sole active ingredient.

### Background Art

Recently according to change of life style and environment, allergic dermatitis, such as bronchial asthma, allergic rhinitis, atopic dermatitis, etc. has rapidly increased.

Nowadays many antiallergic agents are sold. In case of an oral preparation thereof it is anxious for its side effects, such as sleepiness, laziness, etc.

On the other hand, an external preparation comprising a nonsteroidal antiinflammatory agent is used, but it is also anxious for its side effects, such as dermal irritation, contact dermatitis, etc.

Furthermore although steroids for an external application are essential for the therapy of allergic dermatitis, these are not only anxious for their side effects, such as atrophia cutis, steroid flush, angiotelectasis, etc., when repeatedly taken, but also these steroids are transdermally absorbed to migrate to blood and have a possibility to give systemically bad effects.

Acetylsalicylic acid (Hereinafter it may be written as Aspirin.) has a strong analgesic activity, an antifebrile activity and an antirheumatic activity being less in its side effects and being superior in its safety. Therefore, Aspirin has been widely used from of old.

Recently there have been the studies for an application of an external preparation containing acetylsalicylic acid. As a result a composition being superior in transdermal absorption, a new gel-composition or a tape preparation, a plaster improved in shelf life and stability, and a plaster being superior in the drug' absorption and being less in irritation are disclosed in the published patent specifications, etc.

Furthermore, as a new use of acetylsalicylic acid, ointments for treating neuralgia (Japanese Patent Pub. A3-72426), external preparations for treating skin injury (Japanese Patent Pub. A9-235232), a transdermal administration system for treatment of thrombosis and for prophylactic treatment of cancer (Japanese Patent Pub. Tokuhyo 8-504198) are illustrated.

On the other hand in Japanese Patent Pub. A8-208487 it is shown that in regard to the therapeutic method for allergic dermatitis, the dosage of an adrenal cortical hormone can be reduced by admixing acetylsalicylic acid into a preparation containing the adrenal cortical hormone. However the side effects due to repeated (continuous) administrations thereof are still anxious.

### Disclosure of Invention

The present invention relates to provide external preparations which are excellent in a therapeutic effect on allergic dermatitis and are less in their side effects.

The present inventors have earnestly studied and as a result, have found that the preparation prepared by admixing acetylsalicylic acid as the sole active ingredient into the external preparation shows. an excellent therapeutic activity to allergic dermatitis and that side effect thereof reduces comparing with an external preparation containing a steroid. Thus the present invention has been completed.

The external preparation for treating the allergic dermatitis of the present invention was confirmed to show an excellent antiallergic activity in an experimental animal model for type I allergy and an experimental animal model for type IV allergy, as shown in the pharmacological experiments below.

Additionally as a result of comparison between the preparation containing acetylsalicylic acid as the sole active ingredient of the present invention and the steroid ointment containing acetylsalicylic acid disclosed in Japanese Patent Pub. A8-208487, the preparation of the present invention has been found to be superior to the steroid containing acetylsalicylic acid in their side effect.

Examples of allergic dermatitis for which the external preparation of the present invention is used are atopic dermatitis, eczema, contact dermatitis, seborrheic dermatitis, perioral dermatitis, lichen Vidal, nummular eczema, house-wife eczema, phototoxic dermatitis, sting by insects, itching, etc.

Acetylsalicylic acid contained in the external preparation of the present invention is described in the Pharmacopeia of Japan XIII.

The amount of acetylsalicylic acid in the external preparation depends on form of the preparation, but is 0.05-80%, preferably 0.1-70%, more preferably 0.5-50% per total amount by weight. When the amount of acetylsalicylic acid is more than 80% by weight, it is impossible to maintain the physical property as an external preparation. When the amount of acetylsalicylic acid is less than 0.05% by weight, the pharmacological activity of acetylsalicylic acid does not show enough. The amount as more than 80% or less than 0.05% by weight, therefore is not preferable.

The external preparation of the present invention is not limited as far as it is the preparation in which acetylsalicylic acid can be directly applied on the local surface of skin, such as ointments, solutions (e.g. suspensions, emulsions, lotions), cataplasms, tapes, aerosols and external powders (powders for external use).

The amount of acetylsalicylic acid depends on the preparation, but is 0.05-30% by weight in ointments, creams, gels and lotions, is 0.1-20% by weight in cataplasms, is 5-50% by weight in tapes, and is 10-80% by weight in external powders.

As other ingredients except the active ingredient of the preparation of the present invention can be used any ingredient used in the ordinarily external preparation.

In case of ointments, creams, gels and lotions, bases, such as white vaseline (petrolatum), yellow vaseline, lanolin, purified bee wax, cetanol, stearyl alcohol, stearic acid, hydrogenated oil, hydrocarbon gel, polyethylene glycol, liquid paraffin and squalane; solvents or solubilizing agents, such as oleic acid, isopropyl myristate, glycerol triisooctanoate, crotamiton, diethyl sebacate, diisopropyl sebacate, diisopropyl adipate, hexyl laurate, a fatty acid, a fatty acid ester, a plant oil, an aliphatic alcohol and an alcohol; antioxidants, such as a tocopherol derivative, L-ascorbic acid, dibutylhydroxytoluene and butylhydroxyanisole; antiseptics such as p-hydroxybenzoate; humectants, such as glycerin, propylene glycol and sodium hyaluronate; surfactants, such as a polyoxyethylene derivative, a glycerol fatty acid ester, a sucrose fatty acid ester, a sorbitan fatty acid ester, a propylene glycol fatty acid ester and lecithin; thickening agents, such as carboxyvinyl polymer, xanthan gum, carboxymethyl cellulose, sodium carboxymethyl cellulose, hydroxypropyl cellulose and hydroxypropylmethyl cellulose; stabilizers; preservatives; absorption promoters; and other suitable fillers may be added.

In case of cataplasms, tackifiers, such as polyacrylic acid and polyacrylic acid copolymer; crosslinkers, such as aluminum sulfate, aluminum potassium sulfate, aluminum chloride, magnesium alminometasilicate and dihydroxyalminum aminoacetate; thickening agents, such as sodium polyacrylate, polyvinyl alcohol, polyvinylpyrrolidone, gelatin, sodium alginate, carboxymethyl cellulose, sodium carboxymethyl cellulose, hydroxypropyl cellulose and hydroxypropylmethyl cellulose; polyhydric alcohols, such as glycerin, polyethylene glycol (macrogol), propylene glycol and 1,3-butanediol; surfactants such as a polyoxyethylene derivative; perfumes such as ℓ-menthol; antiseptics such as p-hydroxybenzoate; purified water; and other suitable fillers may be added.

In case of tapes, tacking agents, such as a stylene-isoprene-stylene block copolymer and an acrylate resin; tackifier resins, such as an alicyclic saturated hydrocarbon resin, a hydrogenated rosin resin and a terpene resin; softeners, such as liquid gum and liquid paraffin; antioxidants such as dibutylhydroxytoluene; polyhydric alcohols such as polyethylene glycol; absorption promoters such as oleic acid; surfactants such as a polyoxyethylene derivative; and other suitable fillers may be added. In addition a water-absorbable polymer, such as sodium polyacrylate and polyvinyl alcohol, and a small amount of purified water may be added to prepare tape preparations containing water.

In case of aerosols, bases, such as white vaseline (petrolatum), yellow vaseline, lanolin, purified bee wax, cetanol, stearyl alcohol, stearic acid, hydrogenated oil, hydrocarbon gel, polyethylene glycol, liquid paraffin and squalane; solvents or solubilizing agents, such as oleic acid, isopropyl myristate, glycerol triisooctanoate, crotamiton, diethyl sebacate, diisopropyl sebacate, isopropyl adipate, hexyl laurate, a fatty acid, a fatty acid ester, a plant oil, an aliphatic alcohol and an alcohol; antioxidants, such as a tocopherol derivative, L-ascorbic acid, dibutylhydroxytoluene and butylhydroxyanisole; antiseptics such as p-hydroxybenzoate; humectants, such as glycerin, propylene glycol and sodium hyaluronate; surfactants, such as a polyoxyethylene derivative, a glycerol fatty acid ester, a sucrose fatty acid ester, a sorbitan fatty acid ester, a propylene glycol fatty acid ester and lecithin; thickening agents, such as carboxyvinyl polymer, xanthan gum, carboxymethyl cellulose, sodium carboxymethyl cellulose, hydroxypropyl cellulose and hydroxypropylmethyl cellulose, as used in the ointments, the creams, the gels or the lotions; stabilizers; buffering agents; sweetening agents; suspending agents; emulsifying agents; flavors; preservatives; absorption promoters and other suitable fillers, may be added.

In case of external powders, fillers, such as potato starch, rice starch, corn starch, talc and zinc oxide, and other suitable additives may be added.

The external preparations of the present invention can be prepared, for example by well kneading each ingredient, if necessary with a suitable base, in accordance with a usual manner to prepare external preparations.

Thus prepared preparation is applied to the lesion, if necessary.

### Best Mode for Carrying out Invention

The external preparations containing acetylsalicylic acid of the present invention are explained by examples and experimental examples, but the present invention is not limited by these examples.

### Examples 1-10 (Ointments)

According to ingredients indicated in Table 1, hydrocarbon gel and a solvent (oleic acid, Tween 80, crotamiton, diisopropyl adipate, isopropyl myristate, etc.) were dissolved by warming on a water bath, and thereto was added acetylsalicylic acid (Aspirin) to dissolve or well disperse under stirring. Then the mixture was cooled under stirring to prepare ointments.

**Table 1:**

| Ingredients of ointments containing Aspirin | | | | | | | | | | |
|---|---|---|---|---|---|---|---|---|---|---|
| Examples | 1 | 2 | 3 | 4 | 5 | 6 | 7 | 8 | 9 | 10 |
| Ingredients | Ingredient ratio (wt%) | | | | | | | | | |
| Aspirin | 0.1 | 0.5 | 2.0 | 10.0 | 20.0 | 2.0 | 2.0 | 2.0 | 2.0 | 2.0 |
| Oleic acid | - | - | - | - | - | 5.0 | - | - | - | - |
| Tween 80 | - | - | - | - | - | - | 5.0 | - | - | - |
| Crotamiton | - | - | - | - | - | - | - | 5.0 | - | - |
| Diisopropyl adipate | - | - | - | - | - | - | - | - | 5.0 | - |
| Isopropyl myristate | 2.5 | 2.5 | 2.5 | 2.5 | 2.5 | - | - | - | - | 5.0 |
| Hydrocarbon gel | 97,4 | 97.0 | 95.5 | 87.5 | 77.5 | 93.0 | 93.0 | 93.0 | 93.0 | 93.0 |

### Examples 11-15 (Lotions)

According to ingredients indicated in Table 2, Aspirin was added to a warmed oil layer to dissolve or disperse. Separately other ingredients were dissolved in previously warmed purified water, and the oil layer was added thereto under vigorously stirring. The mixture was mixed to homogeneity under gradually cooling to prepare lotions.

**Table 2:**

| Ingredients of lotions containing Aspirin | | | | | |
|---|---|---|---|---|---|
| Examples | 11 | 12 | 13 | 14 | 15 |
| Ingredients | Ingredient ratio (wt%) | | | | |
| Aspirin | 0.5 | 2.0 | 10.0 | 2.0 | 2.0 |
| Crotamiton | 1.0 | 2.0 | 5.0 | - | - |
| Isopropanol | - | - | - | 2.0 | - |
| Diisopropyl sebacate | - | - | - | - | 5.0 |
| Squalane | 3.0 | 3.0 | 3.0 | 3.0 | 3.0 |
| Cetanol | 3.0 | 3.0 | 3.0 | 3.0 | 3.0 |
| Solbitan sesquioleate | 0.5 | 0.5 | 0.5 | 0.5 | 0.5 |
| Polyoxy(20)cetyl ether | 1.5 | 1.5 | 1.5 | 1.5 | 1.5 |
| Propylene glycol | 5.0 | 5.0 | 5.0 | 5.0 | 5.0 |
| Triethanolamine | 0.4 | 0.4 | 0.4 | 0.4 | 0.4 |
| Purified water | 85.1 | 82.6 | 71.6 | 82.6 | 79.6 |

### Examples 16-20 (Gels)

According to ingredients indicated in Table 3, after a water soluble polymer was dissolved on a water bath, Aspirin was dissolved or dispersed in a solvent and these ingredients with other bases were stirred up to homogeneity to prepare gels.

**Table 3:**

| Ingredients of gels containing Aspirin | | | | | |
|---|---|---|---|---|---|
| Examples | 16 | 17 | 18 | 19 | 20 |
| Ingredients | Ingredient ratio (wt%) | | | | |
| Aspirin | 0.1 | 2.0 | 10.0 | 5.0 | 5.0 |
| Crotamiton | 5.0 | 5.0 | 5.0 | 3.0 | - |
| Isopropanol | - | - | - | 3.0 | 5.0 |
| Propylene glycol | 45.0 | 45.0 | 45.0 | 45.0 | 45.0 |
| Polyacrylic acid | 25.0 | 25.0 | 25.0 | 25.0 | 25.0 |
| Triethanolamine | 0.7 | 0.7 | 0.7 | 0.7 | 0.7 |
| Purified water | 24.2 | 22.3 | 14.3 | 18.3 | 19.3 |

### Examples 21-25 (Creams)

According to ingredients indicated in Table 4, after a solid base was dissolved on a water bath, Aspirin dissolved or dispersed in a solvent was added thereto. A watersoluble base was dissolved in water and its warmed solution was added to the mixture. The mixture was kneaded until it became homogenous to prepare creams.

**Table 4 :**

| Ingredients of ointments containing Aspirin | | | | | |
|---|---|---|---|---|---|
| Examples | 21 | 22 | 23 | 24 | 25 |
| Ingredients | Ingredient ratio (wt%) | | | | |
| Aspirin | 0.5 | 2.0 | 10.0 | 5.0 | 5.0 |
| Crotamiton | 2.5 | 2.5 | 2.5 | 5.0 | - |
| Sesame oil | - | - | - | - | 5.0 |
| Diisopropyl sebacate | 2.5 | 2.5 | 2.5 | - | - |
| Cetanol | 9.0 | 9.0 | 9.0 | 9.0 | 9.0 |
| White vaseline | 8.0 | 8.0 | 8.0 | 8.0 | 8.0 |
| Hexyldecanol | 1.0 | 1.0 | 1.0 | 1.0 | 1.0 |
| Polyethylene glycol monostearate | 2.0 | 2.0 | 2.0 | 2.0 | 2.0 |
| Polyoxy(9) lauryl ether | 2.8 | 2.8 | 2.8 | 2.8 | 2.8 |
| Polyoxy(23) cetyl ether | 2.0 | 2.0 | 2.0 | 2.0 | 2.0 |
| Propylene glycol | 12.0 | 12.0 | 12.0 | 12.0 | 12.0 |
| Methylparaben | 0.1 | 0.1 | 0.1 | 0.1 | 0.1 |
| Propylparaben | 0.1 | 0.1 | 0.1 | 0.1 | 0.1 |
| Purified water | 57.5 | 56.0 | 48.0 | 53.0 | 53.0 |

### Examples 26-30 (Tapes)

According to ingredients indicated in Table 5, to a tacking agent consisting of an acrylate resin or a stylene-isoprene-stylene block copolymer were added an alicyclic saturated hydrocarbon resin, liquid paraffin, polybutene, an antioxidant, etc. and the mixture was dissolved in an organic solvent such as toluene etc. under stirring, or the mixture was melted by heating under stirring. Thereto was added Aspirin and the resulting mixture was spread on releasing paper and in case of a solution type, was spread on releasing paper and dried. The releasing paper was laminated on a flexible support to be cut into a desired size to prepare tapes.

**Table 5:**

| Ingredients of tapes containing Aspirin | | | | | |
|---|---|---|---|---|---|
| Examples | 26 | 27 | 28 | 29 | 30 |
| Ingredients | Ingredient ratio (wt%) | | | | |
| Aspirin | 10.0 | 30.0 | 50.0 | 30.0 | 30.0 |
| Isopropyl myristate | - | - | - | - | 5.0 |
| Diisopropyl adipate | - | - | - | 5.0 | - |
| Crotamiton | 5.0 | 5.0 | 7.0 | - | - |
| Acrylate-vinyl acetate copolymer | - | - | - | - | 65.0 |
| Stylene-isoprene-stylene block copolymer | 20.0 | 13.4 | 7.5 | 13.4 | - |
| Alicyclic saturated hydrocarbon resin | 42.0 | 23.5 | 11.7 | 23.5 | - |
| Polybutene | 15.0 | 11.6 | 5.6 | 11.6 | - |
| Liquid paraffin | 7.0 | 15.5 | 17.2 | 15.5 | - |
| Dibutyl hydroxytoluene | 1.0 | 1.0 | 1.0 | 1.0 | - |

### Examples 31-33 (Cataplasms)

According to ingredients indicated in Table 6, a tackifier such as a polyacrylic acid etc. and a thickening agents were dissolved under heating in a polyhydric alcohol such as glycerin etc. After cooling, Aspirin and other fillers were blended to homogeneity and thereto was added a crosslinker to prepare an adhesive gel base. The gel base was spread on a suitable support such as a non-woven fabric to be cut in a desired size to prepare cataplasms.

**Table 6:**

| Ingredients of cataplasms containing Aspirin | | | |
|---|---|---|---|
| Examples | 31 | 32 | 33 |
| Ingredients | Ingredient ratio (wt%) | | |
| Aspirin | 0.5 | 2.0 | 10.0 |
| Polyacrylic acid | 8.0 | 8.0 | 8.0 |
| Sodium polyacrylate | 4.0 | 4.0 | 4.0 |
| Sodium carboxy cellulose | 5.0 | 5.0 | 5.0 |
| Tartaric acid | 1.6 | 1.6 | 1.6 |
| Dihydroxyalminum aminoacetate | 0.07 | 0.07 | 0.07 |
| Glycerin | 34.5 | 33.0 | 25.0 |
| Crotamiton | 2.0 | 2.0 | 2.0 |
| Sesame oil | 1.0 | 1.0 | 1.0 |
| Purified water | 43.33 | 43.33 | 43.33 |

### Examples 34-36 (Powders)

According to ingredients indicated in Table 7, potato starch, zinc oxide and Aspirin were well mixed to prepare powders.

**Table 7:**

| Ingredients of powder containing Aspirin | | | |
|---|---|---|---|
| Examples | 34 | 35 | 36 |
| Ingredients | Ingredient ratio (wt%) | | |
| Aspirin | 20.0 | 40.0 | 80.0 |
| Potato starch | 76.0 | 56.0 | 16.0 |
| Zinc oxide | 4.0 | 4.0 | 4.0 |

### Examples 37-38 (Ointments)

According to ingredients indicated in Table 8, ointments were prepared in the same methods as in Examples 1-5 (Ointments).

**Table 8:**

| Ingredients of ointments containing Aspirin | | |
|---|---|---|
| Examples | 37 | 38 |
| Ingredients | Ingredient ratio (wt%) | |
| Aspirin | 20.0 | 1.0 |
| Isopropyl myristate | 5.0 | 5.0 |
| Hydrocarbon gel | 75.0 | 94.0 |

### Comparative examples 1-4

According to the method described in Japanese Patent Pub. A 8-208487, ointments containing Aspirin and dexamethasone or prednisolone (Comparative examples 1-4 shown in following Table 9 and Table 10) were prepared .

**Table 9:**

| Ingredients of ointments containing Aspirin and dexamethasone | | |
|---|---|---|
| Comparative examples | 1 | 2 |
| Ingredients | Ingredient ratio (wt%) | |
| Aspirin | 20.0 | 1.0 |
| Dexamethasone | 0.025 | 0.025 |
| Propylene glycol | 10.0 | 1.0 |
| Isopropyl myristate | 1.0 | 1.0 |
| Hydrocarbon gel | 68.975 | 96.975 |

**Table 10:**

| Ingredients of ointments containing Aspirin and prednisolone | | |
|---|---|---|
| Comparative examples | 3 | 4 |
| Ingredients | Ingredient ratio (wt%) | |
| Aspirin | 20.0 | 1.0 |
| Prednisolone | 0.25 | 0.25 |
| Propylene glycol | 10.0 | 1.0 |
| Isopropyl myristate | 1.0 | 1.0 |
| Hydrocarbon gel | 68.75 | 96.75 |

Test [A]: Allergic dermatitis is said to be caused due to immediate type allergy (type I allergy) and delayed type allergy (type IV allergy). On the preparation for treating allergic dermatitis of the present invention, homologous passive cutaneous anaphylaxis (homologous PCA) reaction for 48 hours was tested using experimental animal model for type I allergy, and picryl chloride-induced delayed type allergic reaction was tested using experimental animal-model for type IV allergy. The pharmacological effects to type I allergy and type IV allergy were evaluated.

### Experimental example 1: Effect on type I allergy by PCA reaction

Wistar male rats (about 200-250g weight, 10 rats/group) were used in this test. After removal of hairs on back on the rat, each 0.1ml of antiserum of dinitrophenol bound ascaris extract (DNP-As) (factor: 512) diluted 40 times with physiological saline was intracutenously administered at both sides of median line on the skin of the back to passively sensitize. After 48 hours 1ml of Evans blue (0.5%)/physiological saline containing DNP-As (1mg) was intravenously administered to induce PCA reaction. Thirty minutes after induction of PCA reaction, the rat was bled to death and the skin on the back was released. Breadth and length of the blue stained PCA reaction region were measured from inside by a slide caliper and the stained area was calculated. Each 50mg of ointments of Examples 1-5 and the ointment base were applied to regions where the anti-DNP-As serum had been administered, 5 hours before administration of DNP-As. The evaluation of the preparations was indicated by the blue stained area comparing with the control group.

The results are shown in Table 11.

**Table 11**

| Groups | Content of Aspirin (wt%) | Stained area (mm²) | Inhibited rate (%) |
|---|---|---|---|
| Non-treated | - | 115±5 | - |
| Base | 0 | 116±5 | -0.8 |
| Example 1 | 0.1 | 108±4 | 6.1 |
| Example 2 | 0.5 | 91±7 | 20.8 |
| Example 3 | 2.0 | 67±3 | 41.7 |
| Example 4 | 10.0 | 61±3 | 46.9 |
| Example 5 | 20.0 | 75±6 | 38.7 |
| Means ± standard error | | | |

As shown in the above Table 11, the preparations containing Aspirin significantly inhibited homologous PCA reaction for 48 hours comparing with the non-treated control group and the ointment base group, and showed a superior inhibition activity to type I allergy.

### Experimental example 2: Effect on type I allergy by PCA reaction

According to the method of Experimental example 1 using one group consisting of 6 Wistar male rats (about 165-175g weight), evaluation on lotions (Examples 11-13 and base A), gels (Examples 16-18 and base B) and creams (Examples 21-23 and base C) was carried out. In respect to the lotions, the gels and the creams, 5 minutes before administration of DNP-As each preparation (1ml) was spread on the gauze (5cm x 5cm) and it was put or pasted on the region where anti-DNP-As serum was administered. The evaluation of the preparations was indicated by the blue stained area comparing with the control group.

The results are shown in Table 12.

**Table 12**

| Groups | Content of Aspirin (wt%) | Stained area (mm²) | Inhibited rate (%) |
|---|---|---|---|
| Non-treated | - | 255±13 | - |
| Base A | 0 | 216±5 | 15.3 |
| Example 11 | 0.5 | 160±7 | 37.3 |
| Example 12 | 2.0 | 145±7 | 43.1 |
| Example 13 | 10.0 | 141±7 | 44.7 |
| Base B | 0 | 215±12 | 15.7 |
| Example 16 | 0.1 | 202±11 | 20.8 |
| Example 17 | 2.0 | 153±7 | 40.0 |
| Example 18 | 10.0 | 125±7 | 50.1 |
| Base C | 0 | 208±15 | 18.4 |
| Example 21 | 0.5 | 175±12 | 31.4 |
| Example 22 | 2.0 | 152±6 | 40.4 |
| Example 23 | 10.0 | 161±11 | 36.9 |
| Means ± standard error | | | |

As shown in the above Table 12, the lotions, the gels and the creams respectively containing Aspirin, significantly inhibited homologous PCA reaction for 48 hours comparing with the non-treated control group and each the ointment base group, and showed a superior inhibition activity to type I allergy.

### Experimental example 3: Effect on type I allergy by PCA reaction

According to the method of Experimental example 1 using one group consisting of 6 Wistar male rats (about 165-175g weight), evaluation on tapes (Examples 26-28 and base A) and cataplasms (Examples 31-33 and base B) was carried out. In respect to the tapes and the cataplasms, 5 minutes before administration of DNP-As each preparation (5cm x 5cm) was applied to the region where anti-DNP-As serum was administered. The base weight of the tape and the cataplasm was 0.25g/5cm x 5cm and 2.5g/5cm x 5cm, respectively. The evaluation of the preparations was indicated by the blue stained area comparing with the control group.

The results are shown in Table 13.

**Table 13**

| Groups | Content of Aspirin (wt%) | Stained area (mm²) | Inhibited rate (%) |
|---|---|---|---|
| Non-treated | - | 255±13 | - |
| Base A | 0 | 238±13 | 6.7 |
| Example 26 | 10.0 | 151±6 | 40.8 |
| Example 27 | 30.0 | 142±5 | 44.3 |
| Example 28 | 50.0 | 134±10 | 47.4 |
| Base B | 0 | 211±8 | 17.3 |
| Example 31 | 0.5 | 182±9 | 28.6 |
| Example 32 | 2.0 | 159±6 | 37.6 |
| Example 33 | 10.0 | 164±11 | 35.7 |
| Means ± standard error | | | |

As shown in the above Table 13, the tapes and the cataplasms respectively containing Aspirin, significantly inhibited homologous PCA reaction for 48 hours comparing with the non-treated control group and each the ointment base group, and showed a superior inhibition activity to type I allergy.

### Experimental example 4: Effect on picryl chloride-induced delayed type allergic reaction (type IV allergic reaction)

In this experiment male ICR mice (about 30g weight) were used. A solution (0.1ml) of 7% picryl chloride /ethanol solution was applied to the abdomen of mouse where hairs were removed to sensitize. Six days after sensitization a solution (0.02ml) of 1% picryl/ olive oil solution was applied to right auricle to primarily induce. After 24 hours thickness of right and left auricles was measured with a dial thickness gauge and the increased rate of right auricle per left auricle was calculated. The rate was considered as the edema rate. Mice which were well sensitized by the primary induction (one group of 10 mice) were selected. Four days after the primary induction the sensitization was repeated. Six days later, the secondary induction was carried out and thickness of right and left auricles was measured with a dial thickness gauge. Twenty-four hours after the secondary induction the increased rate of right auricle per left auricle was calculated to get the edema rate.

Fifty mg of each ointment (Examples 8, 9 and Comparative example) were applied to the induced region 6 hours and 8 hours after the secondary induction. The evaluation of preparations is indicated by the edema rate comparing with the comparative group.

The results are shown in Table 14.

**Table 14:**

| Picryl chloride-induced delayed type allergic reaction | | | |
|---|---|---|---|
| Groups | Content of Aspirin (% by weight) | Edema rate (%) (%) | Inhibited rate (%) rate (%) |
| Non-treated | - | 84±9 | - |
| Base | 0 | 76±4 | 9.5 |
| Example 8 | 2.0 | 45±6 | 46.4 |
| Example 9 | 2.0 | 50±9 | 40.5 |
| Means ± standard error | | | |

As shown in the above Table 14, the ointments containing Aspirin significantly inhibited Picryl chloride-induced delayed type allergic reaction comparing with the non-treated control group and the ointment base group, and showed a superior inhibition activity to type IV allergy.

Test [B]: The exacerbation of infectious diseases as one of side effects of steroids has been often problematic. On the other hand decrease of the barrier function of skin is indicated as one of causal factors of allergic dermatitis. As being understood from the fact that a lot of bacteria are present in normal skin tissue, it is well known that when steroids are administered to patients suffered from allergic dermatitis, infectious diseases are apt to be caused due to decrease of immunogenecity. As such, using the ointment of the present invention containing only Aspirin as an active ingredient and an ointment containing Aspirin and a steroid described in Japanese Patent Pub. A8-208487), the decrease of the immunogenecity was evaluated by setting the reduction of weight of thymus and adrenal gland as an index.

### Experimental example 5

In this test Wistar male rats (8 weeks old, 6 rats/group) were used. After removal of hairs on the back, the rats were collared not to lick the tested drug (Examples 37, 38 and Comparative examples 1-4) on the back. The tested drug (0.5g/rat/day) was applied to the back in the range of 5cm x 5cm for 7 days. After administration the rat was killed and thymus and adrenal gland were extracted from the rat and their weights were measured.

The results are shown in Table 15.

**Table 15:**

| Thymus weight and adrenal gland weight after administration of each preparation (per body weight (100g)) | | |
|---|---|---|
| Groups Groups | Thymus weight (mg) | Adrenal gland weight (mg) |
| Non-treated | 161±13 | 20.0±1.0 |
| Example 37 | 159±7 | 17.3±0.7 |
| Example 38 | 166±9 | 19.0±1.3 |
| Comparative example 1 | 32±4 | 11.6±0.7 |
| Comparative example 2 | 30±3 | 13.0±1.1 |
| Comparative example 3 | 92±19 | 16.7±0.7 |
| Comparative example 4 | 71±17 | 15.9±1.5 |
| Means ± standard error | | |

As shown in Table 15, the preparations of the present invention (Examples 37 and 38) did not reduce much weight of thymus and adrenal gland comparing with the ointments containing Aspirin and a steroid of the comparative examples. The result shows that the preparation of the present invention does not induce decrease of immunogenecity.

### Possibility of Industrial applicability

The external preparation for treating allergic dermatitis of the present invention contains only Aspirin as an active ingredient and has an excellent activity to the diseases due to allergic dermatitis. Furthermore, the external preparation of the present invention does not reduce weights of thymus and adrenal gland even by continuous applications and therefore, the preparation of the present invention belongs to the drug being less in its side effects. The present invention can provide the external preparation being not only excellently effective to the diseases due to allergic dermatitis, but also being very little in side effect.

## Claims

1. An external preparation for treating allergic dermatitis containing only acetylsalicylic acid as an active ingredient.

2. An external preparation for treating allergic dermatitis consisting of acetylsalicylic acid and fillers.

3. Use of only acetylsalicylic acid as an active ingredient for preparing an external preparation for treating allergic dermatitis.

4. A method for treating a patient suffering from allergic dermatitis comprising transdermally administering a preparation containing only acetylsalicylic acid as an active ingredient to the patient.
